# EUROPEAN PATENT APPLICATION

(11) **EP 3 593 710 A2**
(43) Date of publication of application: **15.01.2020**
(21) Application number: 19192572.6
(22) Date of filing: 15.11.2011
(51) Int. Cl.: A61B 5/00, A61B 18/14, A61B 1/00, A61M 25/00

(54) **CATHETER WITH OPTICAL CONTACT SENSING**

(30) Priority: 16.11.2010 US 94691010
(62) Divisional of application: 11189133.9
(71) Applicant: Biosense Webster (Israel) Ltd., Yokneam, 2066717 (IL)
(72) Inventor: Govari, Assaf, 34400 Haifa (IL); Ephrath, Yaron, 35170 Karkur (IL); Beeckler, Christopher Thomas, Brea, CA California 92821 (US); Papaiannou, Athanassios, Brea, CA California 90066 (US); Garcia, Ariel, Glendora, CA California 91741 (US); Altmann, Andres Claudio, 34614 Haifa (IL)
(74) Representative: Small, Gary James

(57) **Abstract**

A medical probe, including a biocompatible sheath having proximal and distal ends, and having at least one transparent strip between the proximal end and the distal end. The probe also has one or more functional elements positioned within the biocompatible sheath.

## Description

### FIELD OF THE INVENTION

The present invention relates generally to invasive probes, and specifically to an optical contact sensing probe.

### BACKGROUND

A wide range of medical procedures involves placing objects, such as sensors, tubes, catheters, dispensing devices, and implants, within the body. Various types of sensors have been proposed for assessing the quality of contact between a catheter and tissue in the body.

The quality of catheter-tissue contact can be verified, for example, by sensing actual physical contact and/or proximity between the catheter and the tissue. U.S. Patent Application 12/816,492 whose disclosure is incorporated herein by reference, describes a catheter with multiple optical contact sensors integrated along its distal end. Each optical contact sensor comprises a combination of at least one optical emitter, such as a Light Emitting Diode (LED), and at least one respective optical detector (such as a photodiode or a phototransistor) in close proximity to the emitter. At small distances from the tissue, the optical detector senses optical radiation, which is emitted by the optical emitter and reflected from the tissue. The optical detector produces a signal that is indicative of the sensed reflection. As the optical contact sensor comes into physical contact with the tissue, the signal will increase to a maximal level. The signal produced by the optical detector thus gives an indication of the quality of contact between the tissue and the distal end of the catheter.

The description above is presented as a general overview of related art in this field and should not be construed as an admission that any of the information it contains constitutes prior art against the present patent application.

### SUMMARY OF THE INVENTION

An embodiment of the present invention provides a medical probe, including:
a biocompatible sheath having proximal and distal ends, and having at least one transparent strip between the proximal end and the distal end; and
one or more functional elements positioned within the biocompatible sheath.

Typically, each of the one or more functional elements includes an optical contact sensor having an optical emitter, and an optical detector in close proximity to the optical emitter. The optical contact sensor may be configured to detect proximity of the distal end to body tissue, and to verify contact between the distal end and the body tissue. The optical contact sensor typically faces the at least one transparent strip. In one embodiment the probe includes one or more electrodes disposed along the biocompatible sheath which are configured to perform an ablation, and the optical contact sensor is configured to provide an indication for controlling the ablation. The optical contact sensor may be configured to provide a further indication for assessing a quality of the ablation.

There is further provided, according to an embodiment of the present invention, a medical probe, including:
a biocompatible sheath having proximal and distal ends, and having at least one transparent element;
a dielectric substrate which is inserted within the biocompatible sheath;
one or more electronic components positioned on the dielectric substrate; and
one or more printed wiring traces positioned on the dielectric substrate and coupled to each of the one or more electronic components.

The at least one transparent element may include a transparent strip between the proximal end and the distal end of the sheath. In a disclosed embodiment each of the one or more electronic components includes an optical contact sensor having an optical emitter, and an optical detector in close proximity to the optical emitter. The optical contact sensor typically faces the transparent strip. The dielectric substrate may include a flexible printed circuit board material. The one or more electronic components may be positioned on an outer side of the dielectric substrate, and the one or more printed wiring traces may be positioned on an inner side of the dielectric substrate.

There is further provided, according to an embodiment of the present invention, a method, including:
incorporating at least one transparent strip between proximal and distal ends of a biocompatible sheath; and
positioning one or more functional elements within the biocompatible sheath.

There is further provided, according to an embodiment of the present invention, a method, including:
incorporating at least one transparent element between proximal and distal ends of a biocompatible sheath;
inserting a dielectric substrate within the biocompatible sheath;
positioning one or more electronic components on the dielectric substrate;
positioning one or more printed wiring traces on the dielectric substrate; and
coupling the one or more printed wiring traces to each of the one or more electronic components.

### BRIEF DESCRIPTION OF THE DRAWINGS

The disclosure is herein described, by way of example only, with reference to the accompanying drawings, wherein:
Figure 1 is a schematic, pictorial illustration of a medical system implementing optical contact sensing, in accordance with an embodiment of the present invention;
Figure 2A is a schematic, side view illustration of an optical contact sensing probe with a transparent strip, in accordance with an embodiment of the present invention;
Figure 2B is a schematic, cross-sectional view illustration of a of the optical contact sensing probe with the transparent strip, in accordance with an embodiment of the present invention;
Figure 3A is a schematic side view illustration of the optical contact sensing probe with an optoelectronic strip, in accordance with an embodiment of the present invention;
Figure 3B is a schematic side view of the optoelectronic strip, in accordance with an embodiment of the present invention; and
Figure 3C is a schematic top-down view of an inner side of the optoelectronic strip, in accordance with an embodiment of the present invention.

### DETAILED DESCRIPTION OF EMBODIMENTS

### OVERVIEW

Various diagnostic and therapeutic procedures, such as intracardiac electrical mapping and cardiac ablation, use an invasive probe, such as a catheter, whose distal tip is fitted with at least one electrode. The electrode is typically operated when the probe is pressed against intra-body tissue. In these procedures, it is usually important to ascertain the proximity of the probe to a body cavity surface, and to determine when the distal tip of the probe is in contact with the body cavity surface.

In an embodiment of the present invention, functional elements of the probe are encased in a biocompatible sheath, which incorporates one or more transparent strips between proximal and distal ends of the probe. Aside from the transparent strip(s), the biocompatible sheath may be otherwise opaque. Functional elements, such as optical contact sensors can be positioned within the sheath facing the transparent strip (which serves as a window). In some embodiments, multiple optical contact sensors may be positioned within the sheath facing the same transparent strip at different locations along the length of the probe.

Embodiments of the present invention also provide an optoelectronic strip, integrated into the probe, upon which the multiple sensors are mounted. The optoelectronic strip comprises a long, narrow flexible dielectric substrate, such as a flexible printed circuit board material, with the optical contact sensors positioned on an outer side (of the optoelectronic strip), and printed wiring traces positioned along an inner side (of the optoelectronic strip) and coupled to each of the sensors. In some embodiments, the strip may be integrated longitudinally within the biocompatible sheath, with the optical contact sensors facing the transparent strip as described supra.

Embodiments of the present invention, including the biocompatible sheath incorporating the transparent strip and the optoelectronic strip enable optical contact sensing probes to be produced reliably and inexpensively.

### SYSTEM DESCRIPTION

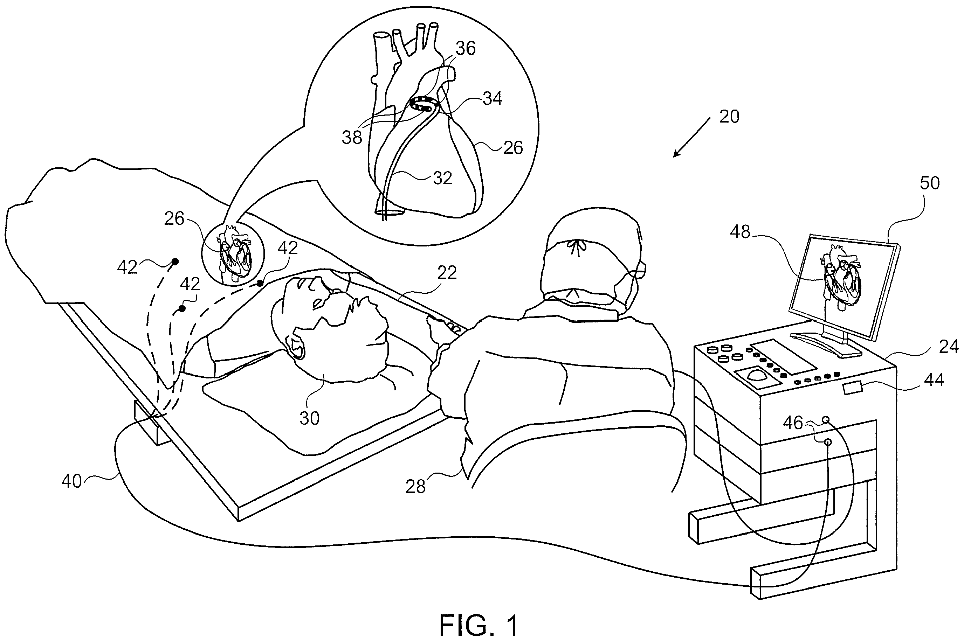

Figure 1 is a schematic, pictorial illustration of a medical system 20 that implements optical proximity sensing, in accordance with an embodiment of the present invention. System 20 comprises an optical contact sensing probe 22, in the present example a catheter, and a control console 24. In the embodiment described hereinbelow, it is assumed that probe 22 is used for diagnostic or therapeutic treatment, such as circumferentially mapping electrical potentials in a pulmonary vein of a heart 26, or performing ablation of the vein tissue. Alternatively, probe 22 may be used, *mutatis mutandis*, for other therapeutic and/or diagnostic purposes in the heart or in other body organs.

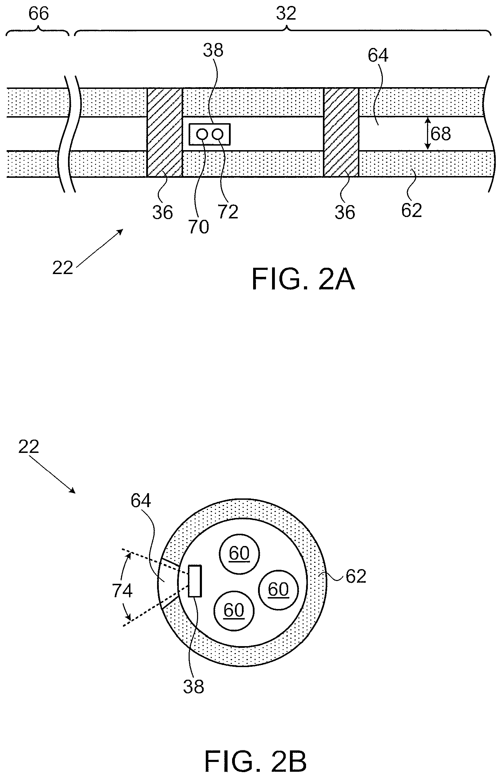

An operator 28, such as a cardiologist, inserts probe 22 through the vascular system of a patient 30 so that a distal end 32 of probe 22 enters a chamber of the patient's heart 26 (e.g., the left atrium). Operator 28 advances probe 22 so that a distal tip 34 (shown here in a "loop" or "lasso" configuration) engages body tissue at desired locations (e.g., vein tissue in the left superior pulmonary vein). Distal tip 34 comprises electrodes 36 and optical contact sensors 38. The configuration of optical contact sensor 38 is shown in greater detail in Figure 2A below. Optical contact sensors are described, for example, in U.S. Patent Application 12/816,492, whose disclosure is incorporated herein by reference. Probe 22 is typically connected by a suitable connector at its proximal end to console 24.

Using signals from the optical contact sensors fitted in probe 22, console 24 determines the quality of contact between distal tip 34 and the vein tissue. The term "quality of contact" refers to actual physical contact between the distal tip and the tissue, as well as proximity of the distal tip to the tissue. In the example of Figure 1, console 24 is also connected by a cable 40 to body surface electrodes, which typically comprise adhesive skin patches 42. Console 24 determines position coordinates of probe 22 inside heart 26 based on the impedance measured between the probe and patches 42. Although system 20 measures position uses impedance-based sensors, other position tracking techniques may be used (e.g., magnetic-based sensors). Magnetic position tracking techniques are described, for example, in U.S. Patents 5,391,199, 5,443,489, 6,788,967, 6,690,963, 5,558,091, 6,172,499 6,177,792, whose disclosures are incorporated herein by reference. Impedance-based position tracking techniques are described, for example, in U.S. Patents 5,983,126, 6,456,864 and 5,944,022, whose disclosures are incorporated herein by reference.

Console 24 comprises a processor 44, which typically comprises a general-purpose computer, with suitable front end and interface circuits for receiving signals from probe 22 and controlling the other components of console 24. An input/output (I/O) communications interface 46 enables console 24 to interact with probe 22 and patches 42. Based on the signals received from probe 22 and from patches 42, processor 44 produces and displays a map 48 showing the position of distal tip 34 in the patient's body, the distance and/or contact indication between the loop and the body tissue, as well as status information and guidance regarding the procedure that is in progress. Map 48 is presented to operator 28 using a display 50. The position of probe 22 may be superimposed on map 48 or on another image of heart 26.

### PROBE WITH A TRANSPARENT STRIP

Figure 2A is a schematic, pictorial illustration of a side view of optical contact sensing probe 22, and Figure 2B is a schematic, pictorial illustration of a cross-section of the probe, in accordance with an embodiment of the present invention. Probe 22 comprises functional elements such as optical contact sensor 38 and tubes 60, which are covered by a biocompatible sheath 62. Sheath 62 is opaque to optical radiation except for a transparent (to optical radiation) strip 64, which is incorporated between a proximal end 66 and distal end 32 of the probe. The proximal and distal ends of the probe are respectively substantially the same as the proximal and distal ends of the biocompatible sheath, so that the terms proximal end 66 and distal end 32 also refer to the corresponding ends of the sheath. Transparent strip 64 is also referred to herein as window 64, and has a width 68.

In the configuration shown in Figure 2A, electrodes 36 are disposed along the length of distal end 32. Electrodes 36 are typically made of a metallic material, such as a platinum/iridium alloy or another suitable material.

Optical contact sensor 38 is positioned within sheath 62 facing window 64, and is typically disposed symmetrically with respect to the window. Optical contact sensor 38 comprises an optical emitter 70 such as a light emitting diode (LED), and an optical detector 72 such as a photodiode or a phototransistor in close proximity to the optical emitter. While the configuration of optical contact sensor 38 shown in Figure 2A comprises one optical emitter 70 and one optical detector 72, the optical contact sensor may be configured to include more than one optical emitter and/or more than one optical detector. Optical contact sensors incorporating optical emitters 70 and optical detectors 72 are described, for example, in U.S. Patent Application 12/816,492, whose disclosure is incorporated herein by reference.

Width 68 of window 64 determines the extent of a field of view 74 of the sensor in the azimuthal direction (i.e., how far optical contact sensor 38 is able to "see" around probe 22). Window 64 may be configured to be narrow, if desired, to ensure that optical contact sensor 38 is sensitive to contact between probe 22 and body tissue only within a desired, narrow angular range (i.e., field of view 74).

Window 64 may be created in the process of producing biocompatible sheath 62 using a coextrusion process, which is a variation of extrusion. During extrusion, an extruder melts a material, which is then conveyed through a die configured to give the final product (e.g., a tubular shaped sheath) a desired profile. The die is designed so that the molten material evenly flows to the product's profile shape. To produce sheath 62 with window 64, two extruders melt and convey a steady volumetric throughput of different biocompatible materials (i.e., for the opaque sheath and the transparent strip, respectively) to a single die which extrudes the materials into the form shown in Figure 2A. Setting width 68 during the coextrusion process enables the production of probe 22 with desired field of view 74.

Although Figure 2A shows probe 22 along a straight probe segment, the same sort of technique may be used in curved elements, such as the curved end of a lasso catheter, as shown in Figure 1. In the example shown in Figure 1, distal tip 34 comprises an adjustable loop fitted with electrodes 36 and optical contact sensors 38. The configuration of the loop enables simultaneous mapping or ablation of circumferential areas of tissue, such as a pulmonary vein.

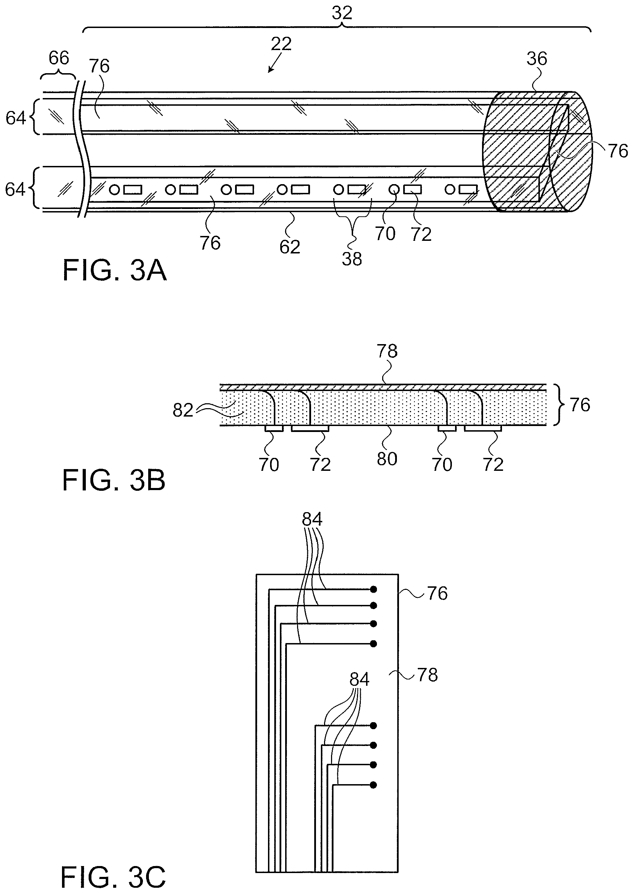

Additionally or alternatively, probe 22 can be produced with multiple, parallel windows 64. For example, in a configuration with two windows 64 located at opposite ends of a probe's diameter (as shown in Figure 3A below), separate optical contact sensors 38 may be positioned and aligned facing each of the two windows. Further alternatively, multiple optical contact sensors 38 may be positioned non-symmetrically with respect to their windows, so that the fields of view of the sensors, while encompassing substantially the same angular width, have different angular coverage. Configuring probe 22 with multiple windows 64 (and corresponding optical contact sensors 38) enables omnidirectional sensing in any direction orthogonal to an axis of the probe, making it possible to sense contact along the length of the probe, regardless of which side of the probe makes contact with the tissue.

### PROBE WITH AN OPTOELECTRONIC STRIP

Figure 3A is a schematic side view illustration of probe 22 with an optoelectronic strip 76, Figure 3B is a schematic side view of the optoelectronic strip, and Figure 3C is a schematic top-down view of an inner side 78 of the optoelectronic strip, in accordance with embodiments of the present invention. Probe 22 incorporates transparent elements, which in the configuration shown in Figure 3A comprise two windows 64 between proximal end 66 and distal end 32 of the probe. Probe 22 comprises optoelectronic strip 76 inserted longitudinally into the probe so that optical contact sensors 38 positioned on an outer side 80 of the optoelectronic strip face one of windows 64.

Optoelectronic strip 76 comprises a long, narrow flexible dielectric substrate 82, such as a flexible printed circuit board material, with optical emitters 70 and optical detectors 72 positioned on outer side 80, and printed wiring traces 84 along inner side 78 that are coupled to each optical emitter 70 and optical detector 72. Optoelectronic strip 76 may be integrated longitudinally into probe 22 during production, either along one side of the probe or wrapped around onto both sides, as shown in Figure 3A. Alternatively, a separate optoelectronic strip 76 can be integrated longitudinally into probe 22 for each window 64.

During operation of the probe, optical emitters 70 emit optical radiation, and optical detectors 72 convey signals to processor 44 indicative of the optical radiation reflecting off the body tissue. Based on the received signals, processor 44 can determine the proximity of distal end 32 to the body tissue, and can verify contact between the distal end and the body tissue.

As discussed supra, probe 22 may be used for ablating vein tissue of heart 26. During an ablation procedure, electrodes 36 spaced along distal end 32 may emit energy, which cauterizes a small amount of the vein tissue. Since cauterized and non-cauterized tissue typically have different reflection properties, optical detectors 72 can be configured to convey different signals, based on the different levels of optical radiation reflecting off the cauterized and the non-cauterized vein tissue. Therefore, processor 44 may use optical sensors 38 to control this and other ablation procedures, as well as to assess a quality of the ablation that has been performed.

Although Figure 3A shows optical emitters 70 and optical detectors 72 positioned on optoelectronic strip 76, electronic components of other types may be positioned on the optoelectronic strip, and are thus considered to be within the spirit and scope of this invention. Examples of electronic components that can be positioned on optoelectronic strip 76 and coupled to printed wiring traces 84 include piezoelectric transducers, capacitive sensors and pressure sensors of other types.

Additionally, the optoelectronic strip described hereinabove assumes that wiring traces 84 and the electronic components (i.e., emitters 70 and detectors 72) are on opposite sides of optoelectronic strip 76. In an alternative embodiment, at least some traces 84 are on the same side (of strip 76) as the electronic components.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A medical probe, comprising:
a biocompatible sheath having proximal and distal ends, and having at least one transparent strip between the proximal end and the distal end; and
one or more functional elements positioned within the biocompatible sheath.

2. The medical probe according to claim 1, wherein each of the one or more functional elements comprises an optical contact sensor comprising an optical emitter, and an optical detector in close proximity to the optical emitter.

3. The medical probe according to claim 2, wherein the optical contact sensor is configured to detect proximity of the distal end to body tissue, and to verify contact between the distal end and the body tissue.

4. The medical probe according to claim 2, wherein the optical contact sensor faces the at least one transparent strip.

5. The medical probe according to claim 2, and comprising one or more electrodes disposed along the biocompatible sheath which are configured to perform an ablation, and wherein the optical contact sensor is configured to provide an indication for controlling the ablation.

6. The medical probe according to claim 5, wherein the optical contact sensor is configured to provide a further indication for assessing a quality of the ablation.

7. A medical probe, comprising:
a biocompatible sheath having proximal and distal ends, and having at least one transparent element;
a dielectric substrate which is inserted within the biocompatible sheath;
one or more electronic components positioned on the dielectric substrate; and
one or more printed wiring traces positioned on the dielectric substrate and coupled to each of the one or more electronic components.

8. A method, comprising:
incorporating at least one transparent strip between proximal and distal ends of a biocompatible sheath; and
positioning one or more functional elements within the biocompatible sheath.

9. The method according to claim 8, wherein each of the one or more functional elements comprises an optical sensor comprising an optical emitter, and an optical detector in close proximity to the optical emitter.

10. The method according to claim 9, wherein the optical sensor faces the at least one transparent strip.

11. A method, comprising:
incorporating at least one transparent element between proximal and distal ends of a biocompatible sheath;
inserting a dielectric substrate within the biocompatible sheath;
positioning one or more electronic components on the dielectric substrate;
positioning one or more printed wiring traces on the dielectric substrate; and
coupling the one or more printed wiring traces to each of the one or more electronic components.

12. The medical probe according to claim 7 or the method according to claim 11, wherein the at least one transparent element comprises a transparent strip between the proximal end and the distal end of the sheath.

13. The medical probe or the method according to claim 12, wherein each of the one or more electronic components comprises an optical contact sensor comprising an optical emitter, and an optical detector in close proximity to the optical emitter.

14. The medical probe or the method according to claim 13, wherein the optical contact sensor faces the transparent strip.

15. The medical probe according to claim 7 or the method according to claim 11, wherein the dielectric substrate comprises a flexible printed circuit board material.

16. The medical probe according to claim 7 or the method according to claim 11, wherein the one or more electronic components are positioned on an outer side of the dielectric substrate, and the one or more printed wiring traces are positioned on an inner side of the dielectric substrate.
